# EUROPEAN PATENT APPLICATION

(11) **EP 4 397 295 A1**
(43) Date of publication of application: **10.07.2024**
(21) Application number: 24176763.1
(22) Date of filing: 20.12.2019
(51) Int. Cl.: A61J 1/14, A61J 9/00, A61M 5/162, B65D 5/74

(54) **ENTERAL NUTRITION CONTAINER CAP AND METHODS OF MAKING AND USING THE CAP**

(30) Priority: 21.12.2018 US 201862783696 P
(62) Divisional of application: 19835300.5
(71) Applicant: Société des Produits Nestlé S.A., 1800 Vevey (CH)
(72) Inventor: ANDREY, Nicolas, 1026 Echandens (CH); GINZBURG, Jean-Daniel, 74160 Feigeres (FR); NASHED, Amir, 1010 Lausanne (CH); PHILIPPE, David Stéphane, 1673 Auboranges (CH)
(74) Representative: Elkington and Fife LLP

(57) **Abstract**

A package closure system may include a package outlet including a first connection element and a cap configured to removably connect to the package outlet using the first connection element and comprising a second connection element. An overcap can be configured to removably connect to the cap using the second connection element. A method for providing a nutritional composition from a container to an enteral line may include: removing an overcap from a cap, the cap is configured to direct a nutritional composition through a channel within the cap, the cap removably connected to the container; connecting the cap sealingly to the enteral line; and applying a pressure to the container to direct the nutritional composition from the container through the channel and into the enteral line.

## Description

### BACKGROUND OF THE INVENTION

The present disclosure generally relates to health and nutrition. More specifically, the present disclosure relates to enteral nutrition container cap devices and methods of making and using the enteral nutrition container caps.

The delivery of nutritional compositions to mammals, such as human patients, that cannot orally ingest food or other forms of nutrition is often of critical importance. For example, enteral bottles and containers having feeding tubes that deposit food directly into the gastrointestinal tract at a point below the mouth are often used to sustain life while a patient is unable, or refuses, to take food orally. Bottles and containers, feeding tubes and other artificial delivery systems and routes can be used temporarily during the treatment of acute medical conditions. For chronic medical conditions, such systems and routes can be used as part of a treatment regimen that lasts for the remainder of a patient's life. No matter the duration of use, these devices often provide the only means for feeding the patient.

Common concerns with the enteral feeding of nutritional compositions are connection quality and spillage when connecting a container to a feeding device. Some nutritional compositions may be dual use, so another common concern is to provide a container that may be used for consumption both orally and as enteral nutrition. A common method to deliver nutritional compositions to a patient is to pour the nutritional composition from a container into an open syringe attached to a feeding tube. Such methods may be difficult for children or patients with fine motor skills challenges. As a result, the nutritional composition may spill out of the open syringe attached to a feeding tube.

### SUMMARY

The present disclosure provides advantages and solutions to problems in existing technologies for connecting enteral containers to enteral feeding tubes. In this regard, a cap may be configured to sealably close a container and may include an integrated connector configured to directly connect to an enteral feeding tube. The cap may be configured to be removed for oral consumption or to provide a connection directly to an enteral feeding tube. The methods and devices disclosed herein can provide an easy to use, multi-functional cap that may be used with containers.

In an embodiment, a container may comprise a pouch, Gualapack^{®}, Doypack^{®}, Smartflex package and/or other container used to store food, food product and/or a beverage.

In an embodiment, a container may be provided with an integral connector for direct connection with an enteral feeding tube. The integral connector may comprise an integrated or separate overcap to close the container. The overcap may be removably or permanently attached to the cap.

In an embodiment, a container provided with an integral connector for direct connection with enteral feeding tubes may eliminate the need for an ancillary medical device such as an adaptor or an administration set. Providing the integral connector for direct connection with an enteral feeding tube may be particularly advantageous when using the nutritional composition for bolus feeding, for example, by eliminating a syringe, adaptor and/or administration set that may be used with such feedings.

In an embodiment, a container provided with an integral connector for direct connection with an enteral feeding tube may provide a tight connection with a feeding tube that may prevent spillage and allow a user to squeeze the container to apply pressure for a faster fluid delivery than without applied pressures. Enabling the squeezing of a container may provide the additional benefit of providing a viscous nutritional composition that may not be able to be delivered through traditional, gravity fed methods.

In an embodiment, a container provided with an integral connector for direct connection to an enteral feeding tube may also be provided with a larger, alternative spout separate from or integral with the integral connector that may be used for oral consumption of the nutritional composition.

In an embodiment, a container provided with an integral connector for direct connection with enteral feeding tubes may provide cost effective manufacturing by attaching to a standard container.

In an embodiment, an integral connector is provided with an air vent to allow for emptying of a rigid container.

In an embodiment, an integral connector is provided with a refilling opening for refilling of a container. The refilling opening may be distinct from an opening configured to attach to an enteral line and/or an opening configured to attach to a container. For example, an integral connector used in a patient hydration application may benefit from a second opening for refilling the container.

In an embodiment, a cap is provided to removably connect to a spout of a container, and the cap is provided without an overcap.

In an embodiment, an integral connector is provided separate from a container, so that the integral connector can be used with known containers and/or a spout of a known container in the field.

In an embodiment, a sealing ring is provided. The sealing ring may be configured to be placed between a spout of a container and a cap to maintain a tight connection between the spout and the cap.

The features and advantages described herein are not all-inclusive and, in particular, many additional features and advantages will be apparent to one of ordinary skill in the art in view of the figures and description. Moreover, it should be noted that the language used in the specification has been principally selected for readability and instructional purposes, and not to limit the scope of the inventive subject matter.

### BRIEF DESCRIPTION OF THE FIGURES

FIG. 1 illustrates a perspective view of an embodiment of a cap according to the present disclosure.
FIG. 2 illustrates a view of a lower portion of an embodiment of the cap according to the present disclosure.
FIG. 3 illustrates a view of an upper portion and the lower portion of an embodiment of the cap according to the present disclosure.
FIG. 4 illustrates a side view of the upper portion and the lower portion of an embodiment of the cap according to the present disclosure.
FIG. 5 illustrates a cutaway view of the upper portion and the lower portion of an embodiment of the cap according to the present disclosure.
FIG. 6 illustrates a cutaway view of the upper portion of an embodiment of the cap according to the present disclosure.
FIG. 7 illustrates a side view of an embodiment of the upper portion of an embodiment of the cap according to the present disclosure.
FIG. 8 illustrates an isometric view of the upper portion of an embodiment of the cap according to the present disclosure.
FIG. 9 illustrates a through-cavity view of the upper portion of an embodiment of the cap according to the present disclosure.
FIG. 10 illustrates an embodiment of a pouch, the cap, and the overcap in a connected configuration according to the present disclosure.
FIG. 11 illustrates an embodiment of the pouch with a spout, the cap, and the overcap in an assembly configuration according to the present disclosure.
FIG. 12 illustrates a detail view of an embodiment of pouch spout threads according to the present disclosure.
FIG. 13 illustrates an embodiment of a cap according to the present disclosure.
FIG. 14 illustrates an embodiment of a cap provided with a spike according to the present disclosure.

### DETAILED DESCRIPTION OF EXAMPLE EMBODIMENTS

Detailed embodiments of devices and methods are disclosed herein. However, it is to be understood that the disclosed embodiments are merely exemplary of the devices and methods, which may be embodied in various forms. Therefore, specific functional details disclosed herein are not to be interpreted as limiting, but merely as a basis for the claims as a representative example for teaching one skilled in the art to variously employ the present disclosure.

As used herein, "about," "approximately" and "substantially" are understood to refer to numbers in a range of numerals, for example the range of -10% to +10% of the referenced number, preferably -5% to +5% of the referenced number, more preferably -1% to +1% of the referenced number, most preferably -0.1% to +0.1% of the referenced number. All numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

The preferred embodiments relate to a cap or connector methods and devices for connecting a container containing nutritional compositions to an enteral feeding device, where the cap or connector may also be used for oral ingestion of the nutritional compositions.

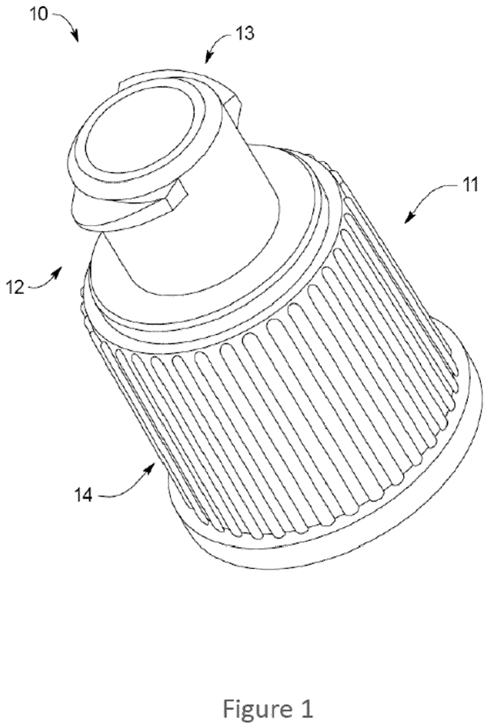

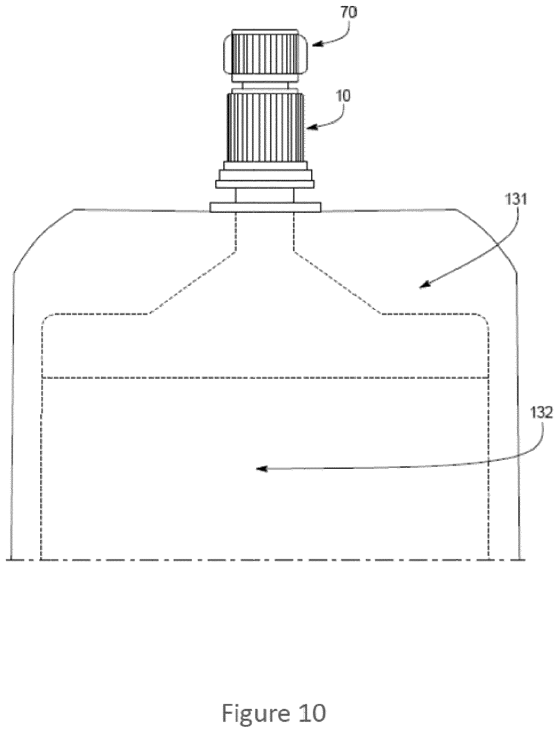

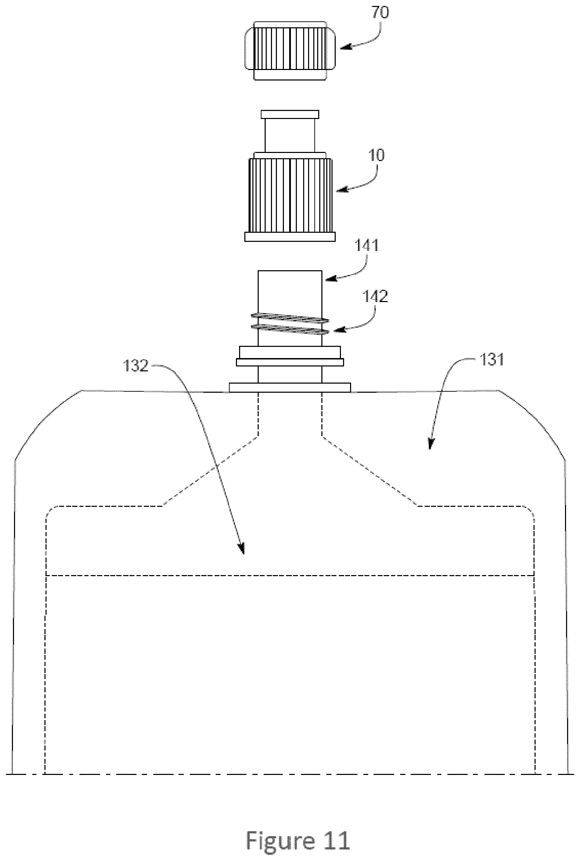

FIG. 1 generally illustrates an embodiment of a cap 10. The cap 10 can comprise: an upper portion 12 for connecting to an external receiving member and/or an overcap 70 as shown in FIGS. 10 and 11; and a lower portion 11 for connecting to a container 131. Referring back to FIG. 1, the external receiving member may be a male ENFit^{®} connector. In an embodiment a female ENFit^{®} connector positioned on the upper portion 12 of the cap 10 may be connected to the male ENFit^{®} connector of the external receiving member. In an alternative embodiment (not shown in Figure 1) the external connector may be a female ENFit^{®} connector, and the upper portion 12 of the cap 10 may be configured as a male ENFit^{®} connector, for connecting to the female ENFit ^{®} connector of the external receiving member. The cap 10 may be provided with outer ridges 14 along an outer circumference of the lower portion 11. The outer ridges 14 may be configured to assist a user when removing, manipulating and/or attaching the cap to other systems or devices. For example, the cap 10 may screw onto other devices containing nutritional product (e.g., a flexible pouch, a SmartFlex Bottle^{®}, a water bottle, a Resource Junior^{®} bottle, an Infrasource^{®} bottle, Tetra^{®} Bottle, other NHSc bottles, and/or other bottles known in the art). Although not shown in FIG. 1, other features of the cap 10 may be provided, for example, larger dimensions, extensions (i.e., wings for grabbing by a user) and/or textured surfaces may be provided as part of the cap 10.

The cap 10 thereby provides direct connection of a container 131 for a nutitional composition with an enteral line feeding tube, without the use of a giving set, administration set or other adaptor.

The outer ridges 14 may be configured perpendicularly to a plane defined by a smallest cross sectional plane of the cap 10, as shown in FIG. 1. Additionally or alternatively, the outer ridges 14 may be crosshatched, textured, and/or comprised of the same or different material as the rest of the cap 10. For example, the outer ridges 14 may be rubber.

The cap 10 may be provided with upper connecting threads 13 extending from the upper portion 12. The upper connecting threads 13 may be configured to attach to an ENFit^{®} connector. In an embodiment, the cap 10 may not be provided with connecting threads 13, and may instead be provided with a clip, snap, plug, fitting and/or other connector for connecting the cap 10 to an external receiving member. More or fewer and/or different connecting threads may be manufactured than the connecting threads 13 shown in FIG. 1. In an embodiment, the cap 10 may only partially comply with ENFit^{®} connector standards. For example, the cap 10 may be provided with a larger bore size than that provided in the ENFit^{®} connector standards to reduce any flow restriction through the cap 10.

The cap 10, as well as connecting components such as the overcap 70, shown in FIGS. 10 and 11, may be manufactured from materials including, but not limited to plastics, sustainable plastics, glass, metal, paper, fiber, carton, edible materials (e.g., fiber, sugar, chews, etc.) and combinations thereof. The cap 10 may be configured to indicate a temperature, a pH, and/or other parameter of a nutritional composition 132, shown in FIG. 10; for example, by manufacturing the cap 10 from a material responsive to the temperature and/or the pH. The color of the cap 10 may correlate to a use for the nutritional composition 132; for example, a cap color may correlate to an embodiment of the nutritional composition 132 where the nutritional composition 132 is optimized to fulfill a specific nutritional requirement. The cap 10 may be manufactured from a flexible material, enabling the cap 10 to be used as a straw. The cap 10 may be manufactured from a transparent material or comprise a transparent section to enable the observation of the nutritional composition 132 flowing through the cap 10.

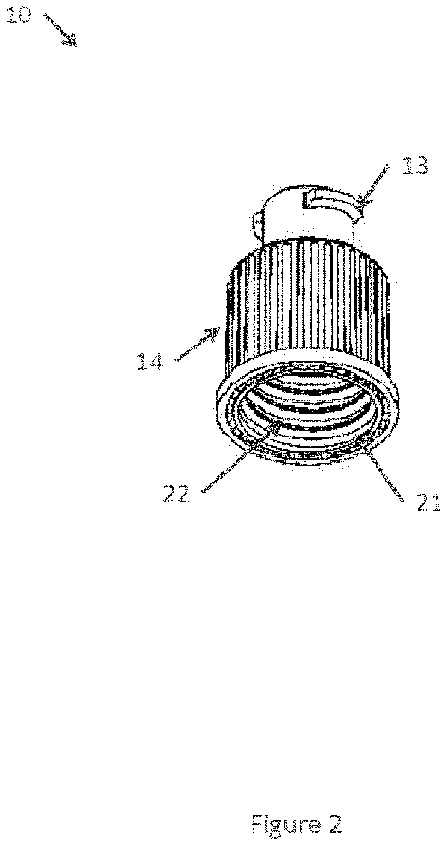

FIG. 2 generally illustrates a view of the lower portion 11 of the cap 10. The upper connecting threads 13 are shown, as well as the outer ridges 14. FIG. 2 shows lower receiving threads 21, which may be positioned within a lower cavity 22 of the cap 10. The lower receiving threads 21 may be configured to attach to the container 131, thereby engaging the container 131 and allowing the nutritional composition 132 to flow from the container 131, into the lower cavity 22, and through other portions of the cap 10.

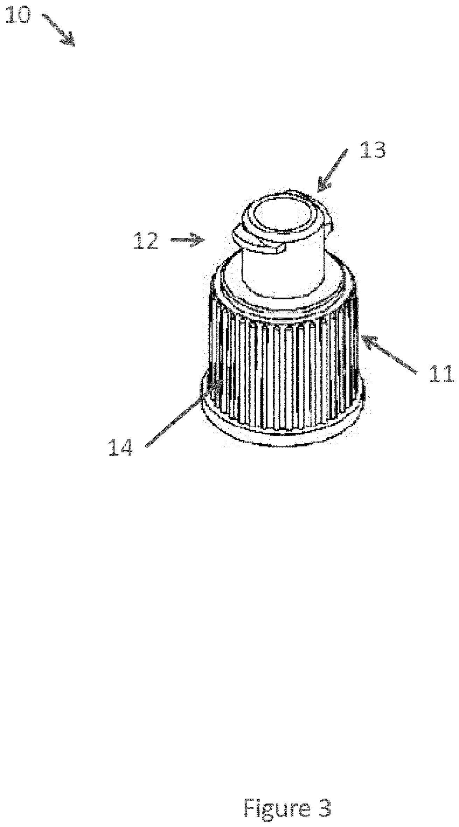

FIG. 3 generally illustrates an isometric view of the cap 10, which may include: the upper portion 12 with the upper connecting threads 13; and the lower portion 11 with the outer ridges 14.

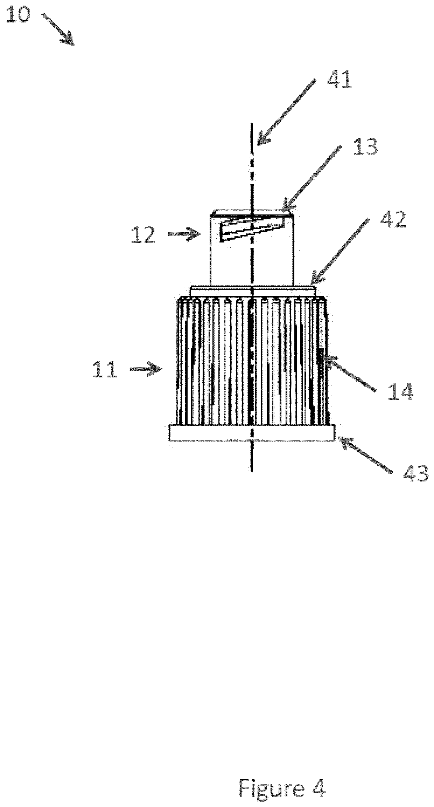

FIG. 4 generally illustrates a side view of the cap 10, which may include: the upper portion 12 with the upper connecting threads 13; and the lower portion 11 with the outer ridges 14. A centerline 41 is shown for reference, which passes through a midline of the generally cylindrical structure of the cap 10. An upper portion base 42 may provide an interface between the upper portion 12 and the lower portion 11. A lower portion base 43 is shown, which can be positioned on the distal end of the lower portion 11 from the upper portion 12.

In an embodiment, a portion of the cap 10 between the upper portion base 42 and the ridges 14 may be elongated to be used as a straw and/or to be more easily manipulated by a user. Such an embodiment may provide better ergonomics and/or better visibility to material flowing through the cap 10 if the cap 10 is manufactured from a transparent material.

In an embodiment, the upper portion 12 of the cap 10 may be retractable, i.e. the upper portion 12 with second connection element 13 may reversible retract, partially or fully, into the cavity 22 of lower portion 11.

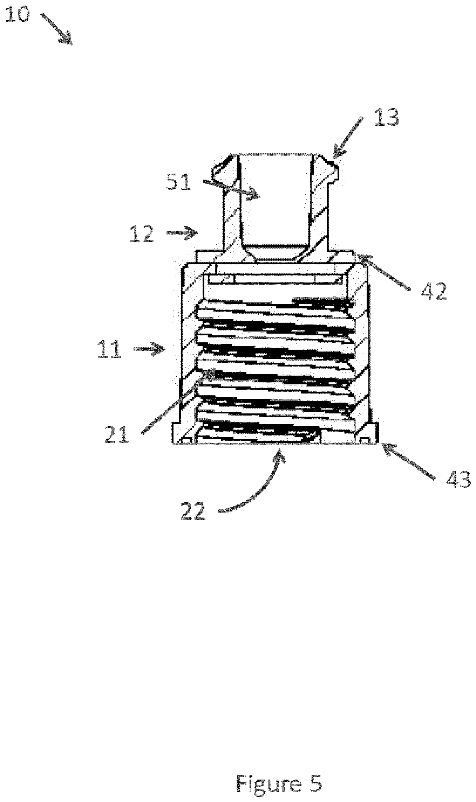

FIG. 5 generally illustrates a cutaway view of the upper portion 12 and the lower portion 11 of the cap 10. As shown, the cap 10 includes an upper cavity 51 that connects to the lower cavity 22, also shown in FIG. 2. Referring back to FIG. 5, the lower receiving threads 21 may extend around an inner circumference of the lower portion 11. The lower receiving threads 21 may extend through the lower portion base 43 to aid the lower portion 11 (e.g., the lower receiving threads 21) in connecting to another connection member.

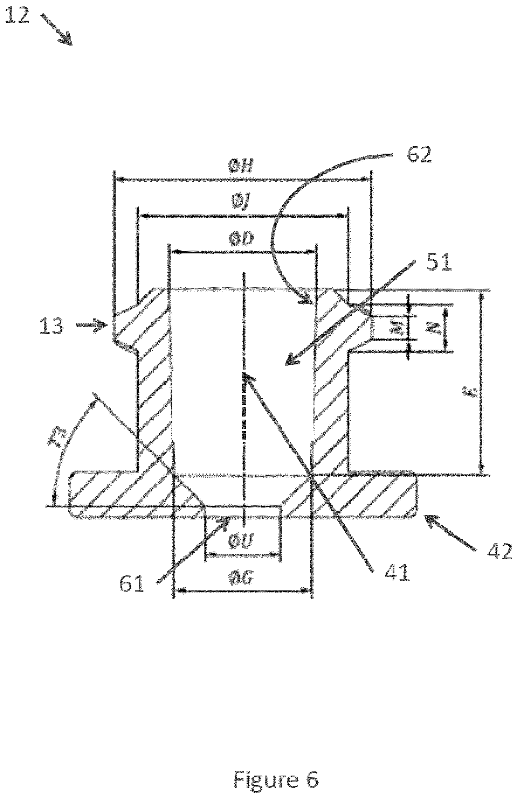

FIG. 6 generally illustrates a dimensioned cutaway view of the upper portion 12 of the cap 10, wherein the upper portion 12 of the cap 10 is adapted for ENFit^{®} compatibility. The upper cavity 51 is shown, which may extend through the upper portion 12 along the centerline 41. The upper cavity 51 may be defined by an upper portion inner wall 62, and have an upper portion inner wall diameter ØD and an upper portion length above base E. A transmission opening 61 may be provided at a distal end of the upper portion 12 from the upper connecting threads 13. The transmission opening 61 may be defined by a transmission opening diameter ØU.

As shown in FIG. 6, the transmission opening diameter ØU may be smaller than an upper portion base inner diameter ØG. In a configuration where the transmission opening diameter ØU is smaller than the upper portion base inner diameter ØG, the transmission opening diameter ØU may create a flow restriction for the nutritional composition 132 flowing through the upper portion 12. In an interface between the upper portion base inner diameter ØG and the transmission opening diameter ØU, a transmission opening chamfer angle T3 may be configured to throttle the flow restriction to adjust flow characteristics, for example flow speed and/or turbulence. In an embodiment, the upper portion base inner diameter ØG and the transmission opening diameter ØU may be equal or about equal, therefore eliminating the flow restriction and resulting in the transmission opening chamfer angle T3 being 90° or about 90°.

The upper portion 12 may comprise an upper portion diameter ØJ, which may correspond to a diameter of the outer walls of the upper portion 12. In an embodiment, the upper connecting threads 13 extend beyond the upper portion diameter ØJ. The upper connecting threads 13 may have an upper connecting threads diameter ØH around the centerline 41, where the upper portion diameter ØJ is smaller than the upper connecting threads diameter ØH.

The upper connecting threads 13 may have a thread crest width M and a thread pitch width N, where the thread pitch width N is preferably a widest point of the upper connecting threads 13. The upper connecting threads 13 may contact the upper portion 12 at the widest point of the upper connecting threads 13. The upper connecting threads 13 may then taper to the most distal portion of the upper connecting threads 13, which may have the thread crest width M. In an embodiment where the upper connecting threads 13 taper to a point, the thread crest width M may be zero or about zero. In an embodiment, the pitch thread width N and the thread crest width M may be equal or about equal, resulting in the upper connecting threads 13 being un-tapered (i.e., straight-cut).

In an embodiment, the relative proportions given in FIG. 6 may be varied to meet different user needs. For example, the upper portion length above base E may be elongated to allow the cap 10 to be used as a straw and/or more easily manipulated by a user. In an embodiment, the upper portion diameter ØJ may be much larger to allow a user to apply more torque to the cap 10 when screwing the cap 10 into a device and/or a component. In an embodiment, the upper portion inner wall diameter ØD may be optimized for a desired flow rate though the cap 10. In an embodiment, the dimensions including at least the thread crest width M, the thread pitch width N and the upper connecting threads diameter ØH may be adapted for connecting to a specific device and/or a specific component.

In an embodiment, the cap 10 may include a strip of foil and/or a seal across the upper portion inner wall diameter ØD. The strip of foil and/or the seal can signal to a user that the cap 10, the container 131, and/or other structure has been tampered with. The strip of foil and/or the seal may also provide added protection against leaks when shipping and/or handling the container 131.

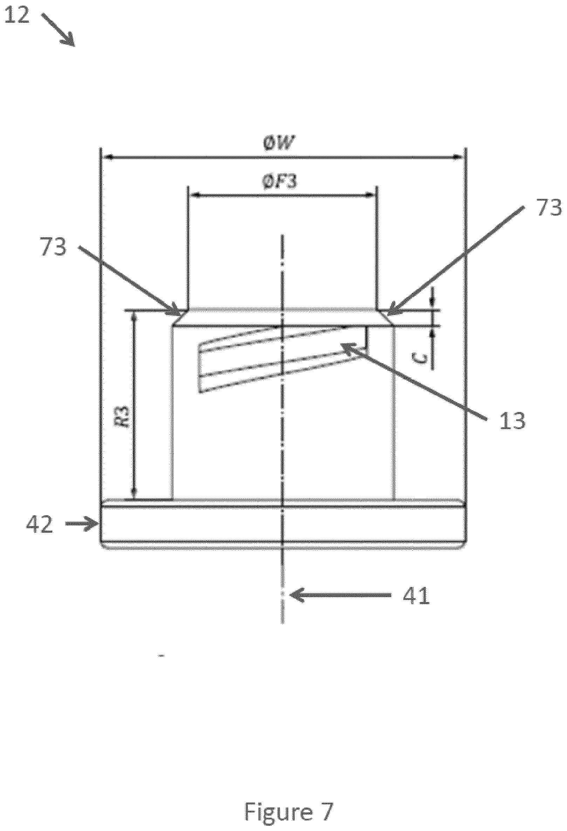

FIG. 7 generally illustrates a side view of an embodiment of the upper portion 12 of the cap 10. The upper portion 12 is shown with upper connecting threads 13. The upper portion 12 may generally be defined by a base width ØW, a top width ØF3 and a length above base R3. The upper portion 12 may include chamfers 73 at an opposite end from the base portion 42 along the centerline 41, and the chamfers 73 may be defined by a chamfer height C.

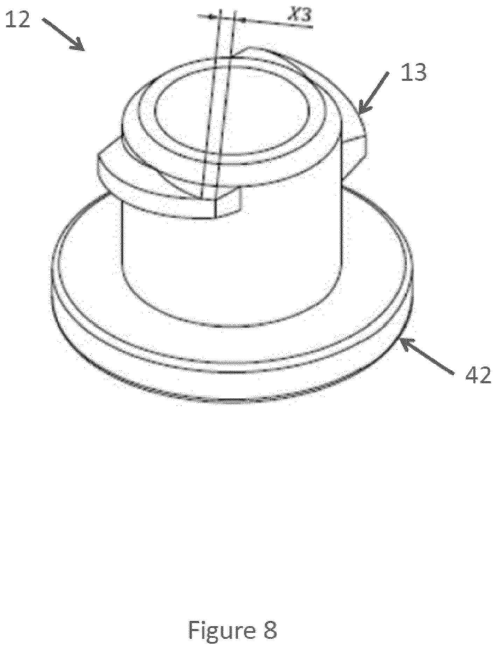

FIG. 8 generally illustrates an isometric view of the upper portion 12 of the cap 10, adapted for ENFit^{®} compatibility. The upper portion 12 may include upper connecting threads 13, which may extend radially outward from the center of the generally cylindrical structure of the upper portion 12. The upper connection threads 13 may be positioned in a plane offset from a plane defined by the upper portion base 42. As the upper connecting threads 13 extend upward, the threads may terminate at an upper connecting thread terminus edge width X3. The upper connecting thread terminus edge width X3 may be provided to prevent misconnection of the cap 10. A configuration of the upper portion 12 may comprise an embodiment of the upper connecting thread terminus edge width X3 where the upper connecting thread terminus edge width X3 is maximized because the contact area provided by a longer embodiment of the upper connecting thread terminus edge width X3 may provide a larger sealing area.

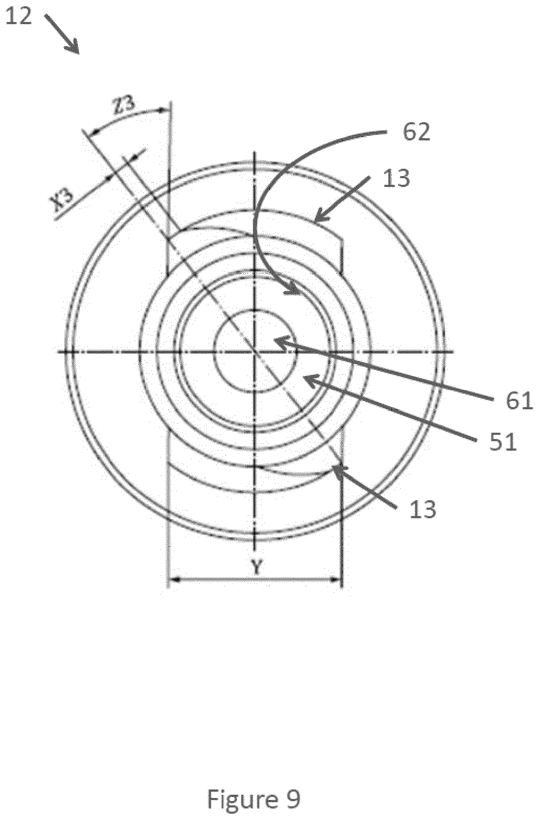

FIG. 9 generally illustrates a through-cavity view of the upper portion 12 of an embodiment of the cap 10. The view shown in FIG. 9 is a view generally down the centerline 41 and shows the upper cavity 51 defined by the upper portion inner wall 62. The upper cavity 51 may terminate into the transmission opening 61, which may provide a path into the lower cavity 22, as shown in FIG. 2. Referring back to FIG. 9, the transmission opening 61 is shown as generally circular, but may be other configurations, for example another geometric shape. The transmission opening 61 may also vary in cross section along the centerline 41, for example the transmission opening 61 may increase and/or decrease in diameter along the centerline 41.

The upper connecting threads 13 may protrude from a part of the upper portion 12 that defines the inner cavity 51. The upper connecting threads 13 are shown with an upper connecting thread width γ which does not extend around the entire circumference of the upper portion 12. In an embodiment, the upper connecting thread width γ may extend around more or less of the circumference of the upper portion 12. An edge of the upper connecting threads 13 may terminate at an upper connecting thread chamfer angle Z3. The upper connecting thread chamfer angle Z3 may be defined as an angle from: a first vector extending from a point defined by the centerline 41 in a plane perpendicular to the centerline 41; and a second vector defined from a locus along the first vector, the second vector extending from the locus.

The upper connecting thread terminus edge width X3 is also shown. The upper connecting thread terminus edge width X3 may be defined as a function of a thread pitch, a thread depth, and a number of threads per unit of distance along the centerline 41.

FIG. 10 generally illustrates an embodiment of a container 131 connected to the cap 10 and with the overcap 70 in place capping the cap 10.

The container 131 is shown as a pouch in the preferred embodiment depicted in FIG. 10, but may also comprise a bag, container, bottle, line, syringe, and/or any other container used to hold and/or transport the nutritional composition 132 and/or fluid. In an embodiment where the container of nutritional composition is deformable, for example when the container 131 is a pouch, a user may squeeze and/or apply pressure to the container 131 to assist in moving the nutritional composition 132 from the container 131 and through the cap 10 or otherwise out of the container 131. In an embodiment, gravity is used to move the nutritional composition 132 from the container 131 and through the cap 10 or otherwise out of the container 131. A configuration where the container 131 is squeezed may move the nutritional composition 132 from the pouch more quickly than using conventional, gravity fed techniques. When the nutritional composition 132 is required for use as a bolus, for example when a bolus of liquid is required, the container 131 may be squeezable and tight connections between the container 131, cap 10, and a patient line are advantageous in reducing spillage of the contents from the container 131.

The cap 10 connects directly to an enteral line feeding tube (not shown), without the use of a giving set, administration set or other adaptor.

In an embodiment, the cap 10 may only be partially screwed onto the container 131. The cap 10 and the container 131 may be configured in a tamper-evident configuration, where a user may irreparably break a seal, plastic retainer, foil, or other mechanism that shows that a package no longer has the original seal and/or is no longer sterile. In an embodiment, the cap 10 and/or the overcap 70 may be attached to the container 131 to break the tamper evident seal.

Although not shown in FIG. 10, other features of the overcap 70 may be provided, for example, larger dimensions, extensions (i.e., wings for grabbing by a user) and/or textured surfaces may be provided as part of the overcap 70.

FIG. 11 generally illustrates an embodiment of the container 131 that is a pouch with a spout 141 comprising pouch spout threads 142, the cap 10, and the overcap 70 in an assembly configuration. The pouch spout threads 142 may be configured circumferentially about the spout 141 and configured to receive the lower receiving threads 21 of the lower portion 11 of the cap 10. In an embodiment, the spout 141 may not be provided with connecting threads 142, and may instead be provided with a clip, snap, plug, fitting and/or other connector for connecting the spout to a corresponding connector element of the lower portion 11 of the cap 10. More or fewer and/or different connecting threads may be manufactured than the connecting threads 142 shown in FIG. 11.

The overcap 70 may be configured to screw into the upper portion 12 by engaging the upper connecting threads 13 (as shown in detail in FIGS. 1-7) with upper receiving threads of the overcap (not shown) to be rotatably attached to the upper portion 12 of the cap 10.

When the overcap 70 is placed over the upper portion 12, a center of the generally cylindrical shape of the overcap 70 may be aligned with the centerline 41, for example the centerline 41 as shown in FIG. 4, to align the overcap 70 with the upper portion 12 for connecting the upper portion 12 to the overcap 70 using the upper connecting threads 13. In an embodiment, the upper connecting threads 13 may not be provided, and the overcap 70 may fit onto the upper assembly using, for example, a snap, a hook, and/or a friction coupling. In an embodiment, the overcap 70 may be a flip-top cap hinged from the upper portion 12 of the cap 10. In an embodiment, the overcap 70 may be a foil strip positioned across the upper portion 12 of the cap 10. In an embodiment the overcap 70 may be configured such that it can be snapped off the upper portion 12 of the cap 10, for example the cap 10 and overcap 70 may be produced as a single element with a weakend portion (such as a zone of thinner material, or a perforated zone) at the interface of the overcap portion 70 and the upper portion 12 of the cap 10, to allow the overcap to be separated (snapped off) from the cap 10.

In an embodiment, a central peg may be included in the overcap 70. The central peg may extend from a closed end of the overcap 70 along a centerline of the overcap toward an open end of the overcap 70. The central peg may aid in closing and/or affixing the overcap 70 to the upper portion 12 of the cap 10. In an embodiment where the upper portion 12 has the strip of foil and/or the seal across the upper portion inner wall diameter ØD (the upper portion inner wall diameter ØD is labeled in FIG. 6), the central peg may be used to puncture the strip of foil and/or the seal. In an embodiment where the upper portion 12 has the strip of foil and/or the seal across the upper portion inner wall diameter ØD, the overcap 70 may not be provided because the sealing function of the overcap 70 may instead be provided by the strip of foil and/or the seal.

The spout 141 may be configured with a larger opening than the opening of the cap defined by the upper cavity 51 of the upper portion 12. The spout 141 may be configured for oral consumption of the nutritional composition 132, while the cap may be configured to be connected to a patient line, thus optionally requiring different sized openings for different uses.

In an embodiment, the spout 141 and or the cap 10 may be sealed using a plastic, metal, foil, or other seal. The seal may indicate whether the container 131 was tampered with if tampering should occur and/or to provided additional sealing protection when shipping the container 131. The seal may be provided to ensure the cap 10, the container 131 (e.g., a pouch), and/or the overcap 70 are sterile.

In an embodiment, the overcap 70, the cap 10, and the container 131 (e.g., a pouch) are provided in a connected manner which may still allow for a user to screw and/or snap the overcap 70, the cap 10, and the container 131 to each other. For example, a retaining line may be provided that connects the cap 10 to the overcap 70 and the container 131. In an embodiment, the overcap 70 may be hinged from the cap 10. In an embodiment, the overcap 70, the cap 10, and the container 131 may all be connected to a connecting member that allows each of the overcap 70, the cap 10, and the container 131 to rotate in a connecting portion of the hinged member. Such a configuration may advantageously prevent misplacing or swallowing of the cap 10, overcap 70, and/or other components of a cap-overcap-container system.

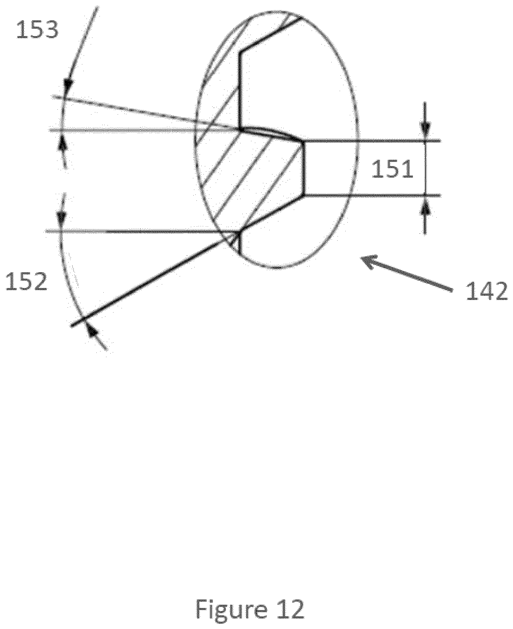

FIG. 12 generally illustrates a detailed cutaway view of the container spout threads 142 of an embodiment of the spout 141. The container spout threads 142 may be defined by a spout thread crest width 151. In an embodiment, the spout thread crest width 151 may be zero or about zero, which results in a thread that comes to a point. The container spout threads 142 may be defined by an upper spout thread half angle 153 and a lower spout thread half angle 152. The upper and lower spout thread half angles 153 and 152 may be defined by an angle of an upper or lower portion of a container spout thread 142 thread tooth from a plane substantially perpendicular to a centerline of a substantially cylindrical embodiment of the spout 141. In an embodiment, the upper spout thread half angle 153 and the lower spout thread half angle 152 are equal or about equal. In an embodiment, the upper spout thread half angle 153 and the lower spout thread half angle 152 are not equal or about equal.

In an embodiment, a method for connecting the container 131 to an enteral line may include removing the overcap 70 from the cap 10, where the cap 10 is configured to flow the nutritional composition 132 through the cap 10. "Removing" means that a user can remove the overcap 70 from the cap 10 without using tools and without damaging the overcap 70 and the cap 10.

The cap 10 may be removably connected to the container 131 and then connected sealingly to the enteral line. "Removably connected" means that that a user can remove the cap 10 from the container 131 without using tools and without damaging the cap 10 and the container 131.

A pressure may be applied to the container 131 to flow the nutritional composition 132 in the container 131 through the cap 10 and into the enteral line. The nutritional composition 132 may be refilled in the container 131 through a refilling opening in the cap 10 and/or through the spout 141. In an embodiment, a seal may be required to be broken when the overcap 70 is connected to the cap 10 prior to connecting the cap 10 sealingly to the enteral line. The method may include disconnecting the cap 10 from the enteral line. The method may include resealing the cap 10 by reconnecting the overcap 70 to the cap 10.

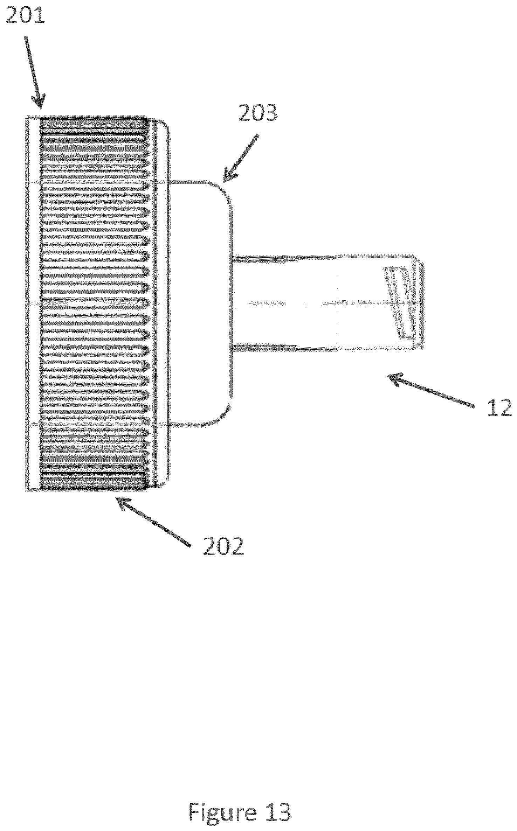

FIG. 13 illustrates an embodiment of the cap 10 with the upper portion 12. In an embodiment, the cap 10 may be configured to connect to containers other than the container 131. For example, in the embodiment according to FIG. 13, the upper portion 12 of the cap 10 may be provided with a Smartflex cap connection 201. The Smartflex cap connection 201 may be configured to connect to a spout of a Smartflex Bottle^{®}. A diameter of the Smartflex cap connection 201 may be larger than a diameter of the upper portion 12. In such an embodiment, a middle portion 203 may be provided. The middle portion 203 may be configured to provide a connection between the relatively large diameter of the Smartflex cap connection 201 with the relatively small diameter of the upper portion 12. In an embodiment, the diameter of the Smartflex cap connection 201 may be closer to the diameter of the upper portion 12, for example within about 10%. In such an embodiment, the middle portion 203 may not be provided, and the Smartflex cap connection 201 may connect directly to the upper portion 12.

In an embodiment, the Smartflex cap connection 201 may be provided with ridges 202. The ridges 202 may be configured perpendicularly to a plane defined by a largest cross sectional plane of the Smartflex cap connection 201, as shown in FIG. 13. Additionally or alternatively, the ridges 202 may be crosshatched, textured, and/or comprised of the same or different material as the rest of the Smartflex cap connection 201. For example, the ridges 202 may be rubber.

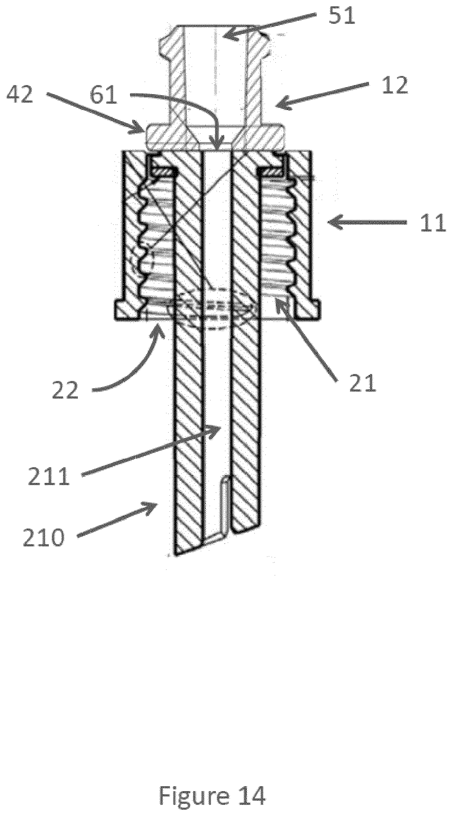

FIG. 14 illustrates an embodiment of the upper portion 12 comprising the upper cavity 51. In the embodiment according to FIG. 14, the upper portion base 42 is configured to be connected to a spike 210. The upper portion base 42 may be configured to connect to the lower portion 11 and the spike 210. In such an embodiment, the lower receiving threads 21 may be configured to connect to a spout, for example the spout 141, and allow for the spike 210 to enter the spout 141. In an embodiment where the spout 141 is sealed, the spike 210 may be configured to break the seal by piercing the seal when the spike 210 enters the spout 141.

The spike 210 may be provided with a spike cavity 211. The spike cavity 211 may be connected to the transmission opening 61. As shown in FIG. 14, a diameter of the spike cavity 211 corresponds to a diameter of the transmission opening 61 and may reduce any flow restriction between the transmission opening 61 and the spike cavity 210. In an embodiment, the lower portion 11 is connected to the spout 141 such that the nutritional composition 132 in the container 131 flows from the container 131, into the spike cavity 211, through the transmission opening 61 and into the upper cavity 51.

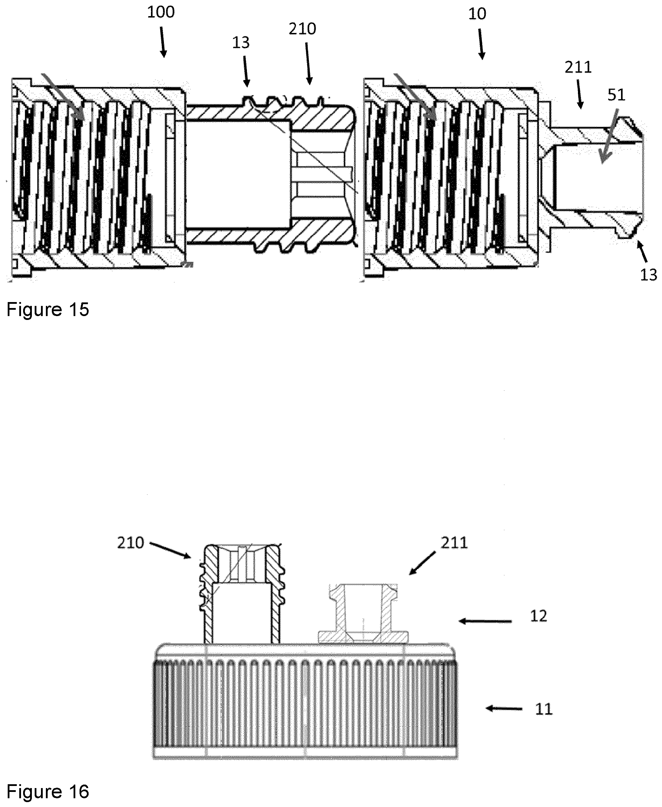

Figure 15 illustrates an embodiment of the cap 10. In the embodiment illustrated in Fugure 15 a second cap 100 is be inserted between the cap 10 and the container 131 (not shown). In the embodiment illustrated in Figure 15 the upper portion 12 of the second cap 100 includes an ENPlus^{®} (cross) port connector (shown as 210) and the upper portion 12 of the first cap 10 includes an ENFit^{®} connector (shown as 211). In the embodiment illustrated in Figure 15 the lower portion of the cap 10 comprises a first threaded connection portion positioned in an inner walled section defining a prximal cavity. This frst threaded connection portion being confirgured for connection with a further threaded portion (shown as 13) on the exterior of the upper portion of the cap 100. In an embodiment, the sequence of the caps 10 and 100 may be inversed.

Figure 16 illustrates another embodiment of the cap 10 with the upper portion 12 and lower portion 11. In an embodiment the upper portion 12 of the cap 10 may include one or more further connection ports / connection elements in addition to the connection element configured for direct connection to the enteral line. For example, in the embodiment according to Figure 16, the upper portion 12 of the cap 10 includes a connection element, shown as 211, configured for direct connection to an enteral line and a second connection element, shown as 210, configured as an ENPlus^{®} (cross) port connector, for connection with an administration set or giving set. The second connection element or connection port may alternatively be configured as another type of connector or port, and may be used as an alternative means to connect the pouch/bottle to a feeding tube, or, for example, as a port allowing introduction of a syringe or tube for addition of a medicament or additive to the nutritional composition, or for introducing water to flush the system.

In an embodiment, a method of making a cap for a container may comprise forming: the cap to have a cylindrical section comprising a proximal end and a distal end; a proximal cavity comprising an inlet opening positioned within the proximal end of the cylindrical section; a distal cavity comprising an outlet opening positioned within the distal end of the cylindrical section; a transmission portion positioned between the proximal cavity and the distal cavity; a first threaded connection positioned around an inner circumference of a walled section defining the proximal cavity; and a second threaded connection positioned around an outer circumference of the distal end of the cylindrical section. The method can comprise injection molding and/or extrusion.

A method of making a package closure system may comprise molding: a package outlet comprising a first connection element; a cap configured to removably connect to the package outlet using the first connection element and comprising a second connection element; and an ENFit^{®} connector and/or overcap configured to removably connect to the cap using the second connection element. In an embodiment, the ENFit^{®} connector and/or the overcap configured to removably connect to the cap are optional.

It should be understood that various changes and modifications to the examples described here will be apparent to those skilled in the art. Such changes and modifications can be made without departing from the spirit and scope of the present subject matter and without diminishing its intended advantages. It is therefore intended that such changes and modifications be covered by the appended claims. Further, the present embodiments are thus not to be limited to the precise details of methodology or construction set forth above as such variations and modification are intended to be included within the scope of the present disclosure. Moreover, unless specifically stated any use of the terms first, second, etc. do not denote any order or importance, but rather the terms first, second, etc. are merely used to distinguish one element from another.

The specification also includes the following embodiments:
[1]: A package closure system comprising:
   a package housing a nutritional composition;
   a spout extending from the package, the spout comprising a first connection element;
   a cap comprising a channel, configured to removably connect directly to the first connection element, and comprising a second connection element, the second connection element configured to connect to an enteral line to position the channel leading from the spout to the enteral line such that the cap indirectly connects the spout to the enteral line by the channel.
[2]: The system according to embodiment [1], wherein the first connection element comprises a first threaded connection element, and
   the second connection element comprises a second threaded connection element.
[3]: The system according to embodiment [1] or [2], wherein the second connection element is an ENFit^{®} connector.
[4]: The system according to any one of embodiments [1] to [3], further comprising an overcap, the overcap configured to close the channel of the cap.
[5]: The system according to embodiment [4], wherein the cap is configured to be disconnectable from the spout,
   wherein the overcap is configured to be disconnectable from the cap, and
   wherein the overcap, the cap, and the spout maintain a physical connection when the cap is disconnected from the spout and the overcap is disconnected from the cap.
[6]: The system according to embodiment [4] or [5], comprising a hinged connection by which the overcap is connected to the cap.
[7]: The system according to any one of embodiments [1] to [6], further comprising an anti-tamper element, the anti-tamper element is configured to be broken when the cap is disconnected from the spout.
[8]: The system according to any one of embodiments [1] to [7], wherein the cap comprises a spike, the spike configured to extend into the spout when the cap is connected to the spout.
[9]: The system according to any one of embodiments [1] to [8], wherein the channel comprises an upper cavity and a lower cavity, and a flow restriction is positioned in the channel and between the upper cavity and the lower cavity.
[10]: The system according to any one of embodiments [1] to [9], wherein the cap comprises a transparent material.
[11]: The system according to any one of embodiments [1] to [10], wherein the cap comprises ridges positioned around a circumference of a substantially cylindrical section of the cap.
[12]: The system according to any one of embodiments [1] to [11], wherein a foil strip is configured to seal the channel of the cap.
[13]: The system according to any one of embodiments [1] to [12], wherein a foil strip is configured to seal the spout of the container.
[14]: A cap comprising:
   a cylindrical section comprising a proximal end and a distal end;
   a proximal cavity comprising an inlet opening positioned within the proximal end of the cylindrical section;
   a distal cavity comprising an outlet opening positioned within the distal end of the cylindrical section;
   a transmission portion positioned between the proximal cavity and the distal cavity;
   a first threaded connection positioned around an inner circumference of a walled section defining the proximal cavity; and
   a second threaded connection positioned around an outer circumference of the distal end of the cylindrical section, said distal end with second threaded connection is configured for direct connection to an enteral line.
[15]: The cap according to embodiment [14], wherein the cap is manufactured from a material responsive to a parameter of a substance that contacts the cap.
[16]: The cap according to embodiment [14] or [15], wherein the outlet opening is sealably covered by a strip of foil.
[17]: A cap comprising:
   a cylindrical section comprising a proximal end and a distal end;
   a proximal cavity comprising an inlet opening positioned within the proximal end of the cylindrical section;
   a distal cavity comprising an outlet opening positioned within the distal end of the cylindrical section;
   a transmission portion positioned between the proximal cavity and the distal cavity;
   a first connection element positioned around an inner circumference of a walled section defining the proximal cavity; and
   a second connection element positioned on the distal end of the cylindrical section, said distal end with second connection element is configured for direct connection to an enteral line.
[18]: A method for providing a nutritional composition from a container to an enteral line, the method comprising:
   connecting the cap sealingly to the enteral line, the cap comprises a channel extending through one side of the cap to the opposite side of the cap, the cap removably connected to the container; and
   applying a pressure to the container to direct the nutritional composition from the container through the channel and into the enteral line, gravity flowing the nutritional composition from the container through the channel and into the enteral line, or a combination thereof.
[19]: The method according to embodiment [18], further comprising removing an overcap from the cap prior to connecting the cap sealingly to the enteral line.
[20]: The method according to embodiment [18], further comprising refilling the container through a refilling opening provided in the cap and distinct from the channel.
[21]: The method according to embodiment [18], further comprising breaking a seal positioned on the cap to seal the cap prior to connecting the cap sealingly to the enteral line.
[22]: The method according to embodiment [18], further comprising:
   disconnecting the cap from the enteral line; and
   sealing the cap by connecting an overcap to the cap.
[23]: The method according to embodiment [18], further comprising removing the cap from a spout positioned on the container, the spout is configured for oral administration of the nutritional composition through the spout.

## Claims

1. A container housing a nutritional composition [132], the container comprising an integral connector for direct connection with an enteral feeding tube, wherein the integral connector is configured to connect to an ENFit^{®} connector.

2. The container according to claim 1, wherein the integral connector comprises a proximal end for connecting to the container and a distal end for connecting to the enteral feeding tube, and wherein connecting threads [13] extend from the distal end and are configured to attach to the ENFit^{®} connector.

3. The container according to claim 2, further comprising an overcap [70], the overcap configured to close the container.

4. The container according to claim 3, wherein the overcap is configured such that it can be snaped off the distal end.

5. The container according to claim 4, wherein the overcap is a single element with a weakened portion at the interface of the overcap and the distal end.

6. The container according to any of claims 1 to 6, wherein the integral connector has a larger bore size than that provided in the ENFit^{®} connector standards.

7. The container according to any of claims 1 to 6, wherein the container is deformable.

8. The container according to claim 7, wherein the container is a pouch.

9. The container according to any of claims 2 to 8, wherein the integral connector further comprises outer ridges along an outer circumference of the proximal portion.

10. The container according to any of the preceding claims, wherein the integral connector further comprises wings for grabbing by a user.

11. The container according to any of the preceding claims, wherein the integral connector is configured to connect to a male ENFit^{®} connector.
